# EUROPEAN PATENT APPLICATION

(11) **EP 2 554 161 A1**
(43) Date of publication of application: **06.02.2013**
(21) Application number: 11176276.1
(22) Date of filing: 02.08.2011
(51) Int. Cl.: A61K 9/107, A61K 38/36, A61K 47/34, A61K 9/00, A61P 7/04

(54) **Pharmaceutical composition comprising factor VII encapsulated in micelles**

(71) Applicant: LFB Biotechnologies, 91940 Les Ulis (FR); NanoCarrier Co., Ltd., Kashiwa-shi, Chiba 277-0882 (JP)
(72) Inventor: Perret, Gérald, F-94600 CHOISY LE ROI (FR); Chtourou, Sami, F-78990 ELANCOURT (FR); Bihoreau, Nicolas, F-91400 ORSAY (FR)
(74) Representative: Le Coupanec, Pascale A.M.P.

(57) **Abstract**

The invention relates to a pharmaceutical composition comprising factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the goup consisting of histidine, lysine, aspartic acid and glutamic acid residues, wherein a part of said amino acid residues is substituted with a hydrophobic group.

## Description

### FIELD OF THE INVENTION

The present invention relates to the field of pharmaceutical compositions comprising factor VII as active ingredient.

### PRIOR ART

Factor VII (FVII) is a vitamin K-dependent glycoprotein which, in its activated form (FVIIa), participates in the coagulation process by activating factor X and factor IX in the presence of calcium and of tissue factor. FVII is secreted in the form of a single peptide chain of 406 amino acid residues, the molecular mass of which is approximately 50 kDa. FVII comprises four distinct structural domains: the N-terminal γ-carboxylic (Gla) domain, two epidermal growth factor-like (EGF-like) domains and one serine protease domain. The activation of FVII to give FVIIa is characterized by cleavage of the Arg152-Ile153 (Arginine 152-Isoleucine 153) bond. FVIIa is therefore composed of a light chain of 152 amino acids, having a molecular mass of approximately 20 kDa, and of a heavy chain of 254 amino acids, having a molecular mass of approximately 30 kDa, linked to one another by a single disulphide bridge (Cysteine 135-Cysteine 262).

FVII/VIIa is used in the treatment of patients suffering from haemophilia, who exhibit a deficiency in factor VIII (haemophilia type A) or in factor IX (haemophilia type B), and also patients who exhibit other coagulation factor deficiencies, for example a hereditary FVII deficiency. FVII/VIIa is also recommended in the treatment of cerebral strokes.

Thus, FVII/FVIIa is used mainly for patient treatments over a very long period of time, which involves giving repeated injections at time intervals which are at most weekly. In practice, a majority of patients treated with factor VII receive two to three weekly injections of FVII/VIIa, which causes considerable inconvenience.

Relatively high doses of FVII/VIIa, and also frequent intravenous administrations, are necessary in order to achieve and preserve the desired therapeutic or prophylactic effect. This treatment therefore remains both restrictive for the patients and very expensive.

FVII/VIIa is also commonly administered to patients who must undergo a surgical procedure. Usually, a first administration of FVII/VIIa, termed "loading dose", for example from 30 to 40 IU/kg, is carried out approximately one hour before the surgical act, and then one or more subsequent injections may be given during the surgical operation and, where appropriate, in the hours or days which follow the surgical operation, in order to maintain the plasma FVII/VIIa level within the accepted norms.

It has been shown that FVII/VIIa is a protein that is sensitive to proteolytic cleavage leading to the formation of many degradation products devoid of coagulant activity (atypical cleavages). Atypical cleavages can occur at various stages of the production process, but also during storage of FVII/VIIa. The degradation products have been observed both for plasma-derived FVII/VIIa, and also for FVII/VIIa produced by genetic recombination. Atypical cleavages can occur before the activation of FVII to give FVIIa, for example during the production and purification of FVII, during the activation step itself or during the purification and/or storage of the activated product (FVIIa).

In order to improve the quality of prophylactic and therapeutic treatments with FVII/FVIIa, it would be advantageous to be able to have pharmaceutical compositions allowing improved FVII/VIIa bioavailability, compared with the bioavailability of the FVII/VIIa of known pharmaceutical compositions. It would in particular be advantageous to have compositions in which the FVII/VIIa has great stability and is less susceptible to the various degradations than in the known compositions.

In particular, it will be advantageous to have FVII/VIIa formulations which make it possible to increase the stability of FVII/FVIIa over time in the organism, and to allow the controlled release of FVII/FVIIa over time, in such a way as to increase the gap between two injections, and thus (i) to improve the quality of life of the patients receiving a chronic treatment and (ii) to facilitate the work of practitioners at the time of a surgical operation, for example by reducing or avoiding the recourse to several injections of FVII/FVIIa during a surgical procedure.

Solutions have already been envisaged in the prior art for improving the bioavailability of FVII/VIIa, for instance encapsulation thereof in liposomes.

Pharmaceutical compositions have also been described in which the FVII/VIIa is non-covalently bound to colloidal particles comprising approximately 1 to 20% in mole of an amphipathic lipid derivatized with a biocompatible hydrophilic polymer. European patent N° EP 1 633 440 describes this type of pharmaceutical composition which comprises FVII/VIIa encapsulated in colloidal particles formed from "PEGylated" liposomes.

There is a need in the prior art for alternative or improved pharmaceutical compositions comprising FVII/FVIIa, compared with the known pharmaceutical compositions.

### SUMMARY OF THE INVENTION

The present invention provides a pharmaceutical composition comprising factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of from histidine, lysine, aspartic acid and glutamic acid residues, wherein a part of said amino acid residues is substituted with a hydrophobic group.

In the said block copolymer molecules, the hydrophilic polymer segment is covalently bound to the hydrophobic polymer segment.

As used herein, polyalkylene glycol is of the following formula (A):

H-[O-R]z-OH (A),

wherein:
- R is a linear alkyl group having from 1 to 4 carbon atoms, and
- z is an integer ranging from 10 to 2500, preferably from 50 to 500.

In some embodiments, R consists of an ethyl group and z ranges from 200 to 300.

In some embodiments of said pharmaceutical composition, the polyalkylene glycol consists of a polyethylene glycol, preferably a polyethylene glycol having a molecular weight ranging from 10 kDa to 12 kDa.

In some embodiments of said pharmaceutical composition, the mass ratio between the factor VII and the copolymer ranges from 1:100 wt:wt to 50:100 wt:wt, preferably from 5:100 wt:wt to 20:100 wt:wt.

In some embodiments of said pharmaceutical composition, the factor VII is chosen from (i) a non-recombinant factor VII purified from plasma and (ii) a recombinant factor VII, where appropriate a recombinant factor VII modified with one or more substitutions or deletions of an amino acid.

The present invention also relates to a pharmaceutical composition comprising factor VII encapsulated in micelles formed from block copolymer molecules containing (i) a hydrophilic polymer segment and (ii) a hydrophobic polymer segment consisting of a polyamino acid, which composition, when it is administered parenterally, is suitable for generating a pharmacokinetic profile having one or more of the following characteristics:
- an AUC (area under the curve) value which is of 10% or more higher than the AUC value obtained with a comparative composition wherein the same amount of FVII is not encapsulated,
- an MRT (mean residence time) value of at least 2 hours,
   and
- a CL (clearance) value of at most 200ml/kg/h.

The values of the MRT and CL should be obtained from seven-week-old rats of the Wistar strain.

As used herein, an AUC value of 10% or more higher than the AUC value of a comparative composition encompasses an AUC value that is 20%, 30%, 40%, 50%, 60%, 70%, 80%, 90%, 100%,or more than 100% higher than the AUC value of the said comparative composition. It has been found that in some embodiments a pharmaceutical composition according to the invention has, for a given amount of FVII/FVIIa comprised therein, an AUC value of twice (100% higher) the AUC value of a comparative composition comprising the same given amount of FVII/FVIIa but in the absence of micelles.

The invention also relates to a factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for use thereof as a medicament.

This invention also relates to a factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for the treatment of coagulation disorders.

### DESCRIPTION OF THE FIGURES

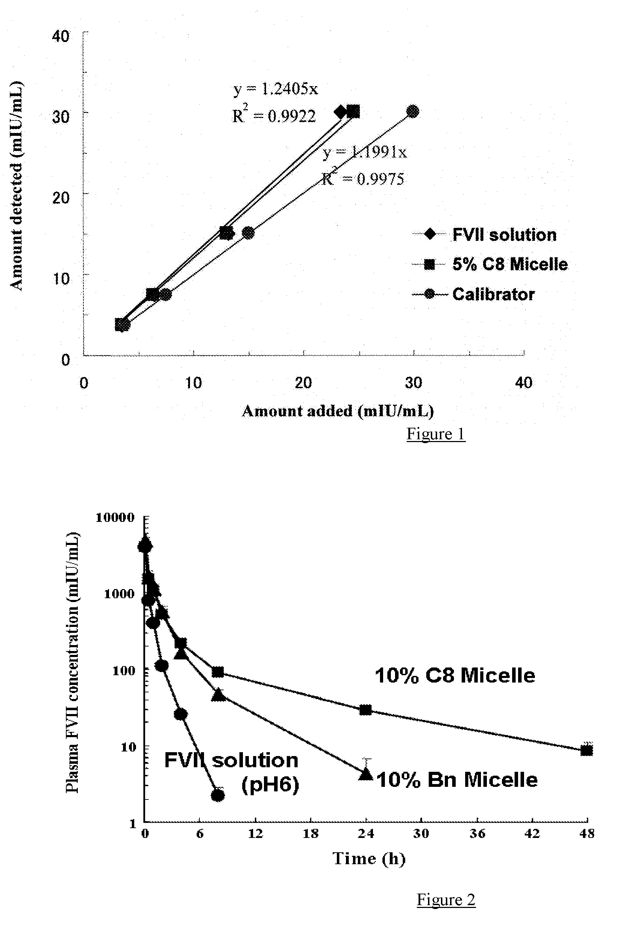

**Figure 1** illustrates the standard curves for determination of the amount of FVII detected respectively in a micelle-free composition containing recombinant human FVII and a composition of FVII encapsulated in micelles, compared with the amount of FVII initially incorporated in order to produce each of the compositions. Along the x-axis: the amount of FVII/VIIa initially added, expressed as milli-international units per millilitre (mIU/ml). Along the γ-axis: the amount of FVII/VIIa detected, expressed as milli-international units per millilitre (mIU/ml).
**Figure 2** illustrates the recombinant human FVII plasma concentration profiles obtained respectively with (i) a micelle-free FVII composition, (ii) a composition of FVII incorporated into micelles of C8-grafted copolymer with a ratio between the FVII and the copolymer of 10:100 and (iii) a composition of FVII incorporated into micelles of Bn-grafted copolymer with a ratio between the FVII and the copolymer of 10:100. Along the x-axis: time elapsed following the injection of the composition, expressed in hours. Along the γ-axis: the plasma FVII/VIIa concentration measured, expressed in milli-international units per millilitre (mIU/ml).

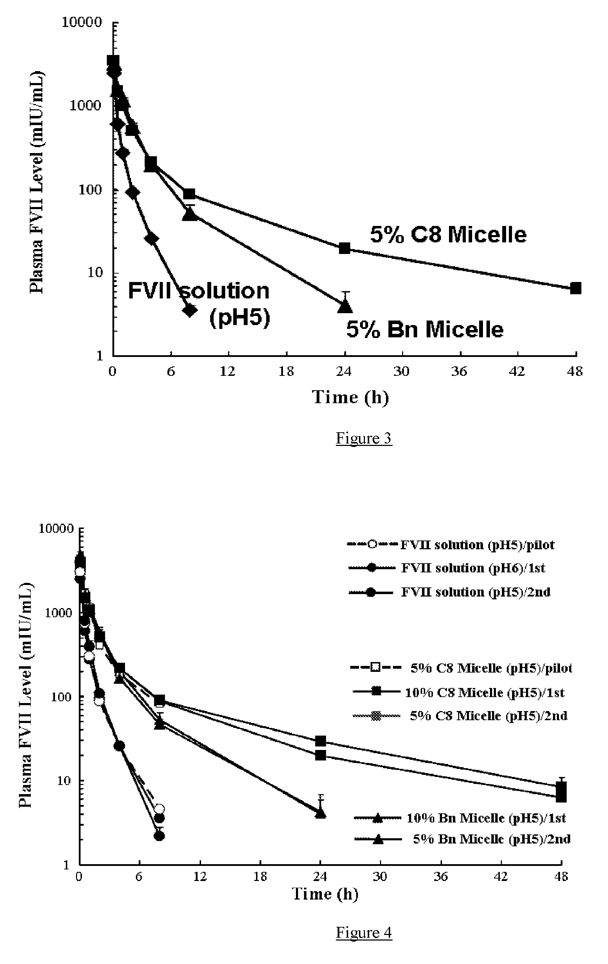

**Figure 3** illustrates the recombinant human FVII plasma concentration profiles obtained respectively with (i) a micelle-free FVII composition at pH 5, (ii) a composition of FVII incorporated into micelles of Bn-grafted copolymer with a ratio between the FVII and the copolymer of 5:100 and (iii) a composition of FVII incorporated into micelles of C8-grafted copolymer with a ratio between the FVII and the copolymer of 5:100.
**Figure 4** illustrates the recombinant human FVII plasma concentration profiles obtained from the FVII solutions and FVII encapsulated micelles being illustrated in the figures 2 and 3.

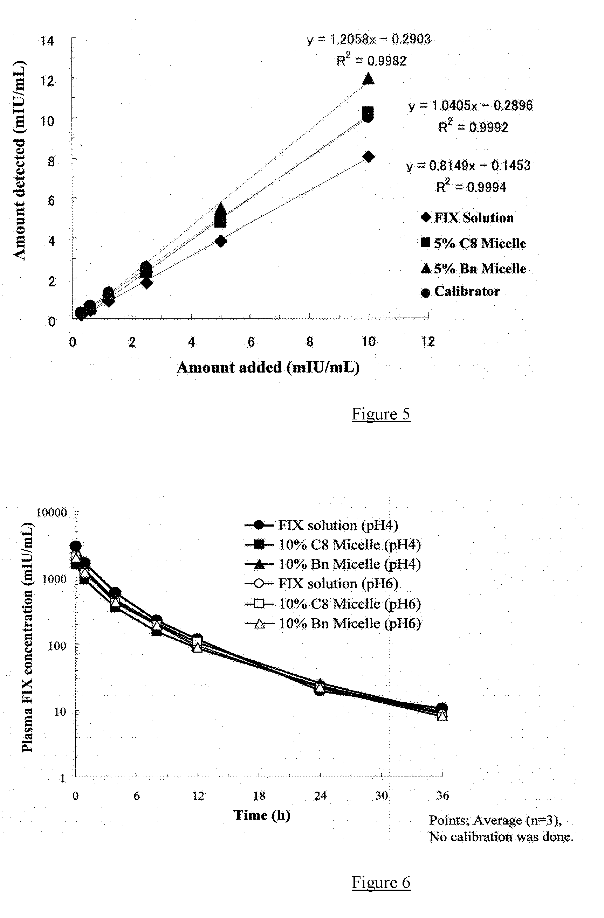

**Figure 5** illustrates the standard curves for determination of the amount of FIX detected respectively in a micelle-free composition containing human FIX and a composition of FIX encapsulated in micelles, compared with the amount of FIX initially incorporated in order to produce each of the compositions. Along the x-axis: the amount of FIX initially added, expressed in milli-international units per millilitre (mIU/ml). Along the γ-axis: the amount of FIX detected, expressed in milli-international units per millilitre (mIU/ml).
**Figure 6** illustrates the human FIX plasma concentration profiles obtained respectively with (i) a micelle-free FIX composition, (ii) a composition of FIX incorporated into micelles of C8-grafted copolymer with a ratio between the FIX and the copolymer of 10:100 and (iii) a composition of FIX incorporated into micelles of Bn-grafted copolymer with a ratio between the FIX and the copolymer of 10:100, all these three solutions being tested at pH=4 and pH=6. Along the x-axis: time elapsed following the injection of the composition, expressed in hours. Along the γ-axis: the plasma FIX concentration measured, expressed in milli-international units per millilitre (mIU/ml).

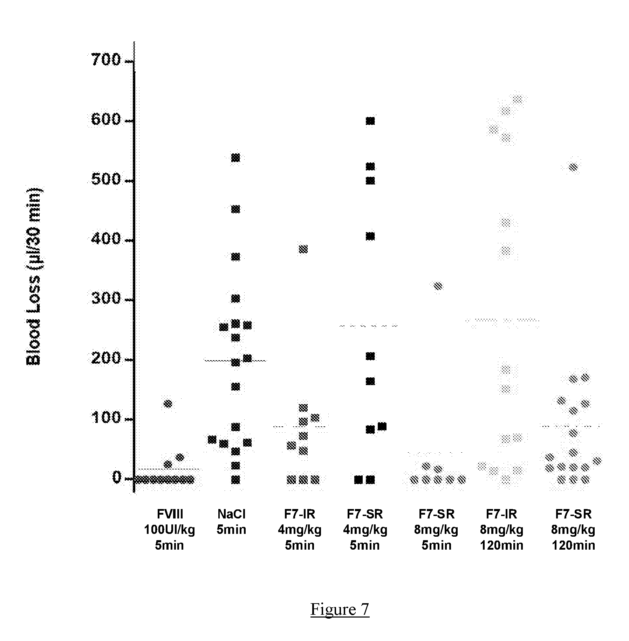

**Figure 7** illustrates the effect of FVII, NaCl 0.9%, F7-IR and F7-SR on hemophilia A mice blood loss. Dots represent individual blood loss and lines mean values.

### DETAILED DESCRIPTION OF THE INVENTION

Surprisingly, it has been shown according to the invention that FVII/FVIIa may be successfully incorporated into micelles of a block copolymer comprising blocks of a specific type containing, in the said block copolymer molecule, a hydrophilic polymer segment and a hydrophobic polymer segment.

It is specified that in the said block copolymer molecule, the hydrophilic polymer segment is covalently bound to the hydrophobic polymer segment.

It has also been shown according to the invention that the incorporation of FVII/FVIIa into micelles of a copolymer comprising blocks of a specific type containing, in the said block copolymer molecule, a hydrophilic polymer segment and a hydrophobic polymer segment makes it possible to significantly increase the bioavailability of the FVII/FVIIa *in vivo.*

In particular, it has been shown in the examples that micelles of this copolymer comprising blocks of a specific type cause controlled release over time of the FVII/FVIIa initially incorporated, and efficiently protect the FVII/VIIa against degradation in the bloodstream.

The present invention provides a pharmaceutical composition comprising factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group.

As used herein, "a part" of said amino acid residues means that one amino acid residue or more of the said polyamino acid has a side chain which is substituted with a hydrophobic group. Preferably, the "part" of substituted amino acid residues ranges from more than 10% to less than 100% of the amino acid residues comprised in the said polyamino acid. In some embodiments, the "part" of substituted amino acide residues ranges from 40% to less than 100%, which includes from 70% to less than 100% of the amino acid residues comprised in the said polyamino acid.

Specific embodiments (i) of the hydrophilic segment consisting of a polyalkylene glycol and (ii) of the hydrophobic segment consisting of a polyamino acid are detailed below, with the description of the block copolymer of formula (I).

It is specified that, preferably, the hydrophobic polyamino acid segment consists of a poly-homo-amino acid chain consisting, for example, of residues of L-amino acids, of D-amino acids or of racemic mixtures thereof. The hydrophobic segment may consist of a polyamino acid chain chosen from a poly(aspartic acid), a poly(glutamic acid), a poly(histidine), a poly(lysine), a poly(aspartic acid ester), a poly(glutamic acid ester), a poly(histidine ester), a poly(lysine ester), a partial hydrolysate of poly(aspartic acid ester), a partial hydrolysate of poly(glutamic acid ester), a partial hydrolysate of poly(histidine ester), or else a partial hydrolysate of poly(lysine ester). The term "partial hydrolysate" of a poly(amino acid ester) is intended to mean that the poly(amino acid) is only partially esterified, i.e. fewer than all of the amino acid residues are esterified.

The hydrophobic segment of said block copolymer above preferably comprises a number of amino acid residues ranging from 10 to 100 amino acid residues, for example from 20 to 75 amino acid residues, which includes from 30 to 50 amino acid residues.

Preferably, in an amino acid ester residue that may be contained in the hydrophobic segment of a block copolymer as defined above, the amino acid residue is esterified with a hydrophobic group. Said hydrophobic group is preferably selected from the group consisting of an alkyl chain containing from 5 to 12 carbon atoms and a benzyl group. Preferably, said alkyl chain consists of an alkyl chain which is unsubstituted or else substituted with one or more alkyl chains containing from 1 to 3 carbon atoms.

It is specified that the general family of block copolymers which are used for the production of a pharmaceutical composition is known in the art. To the applicant's knowledge, these block copolymers have, however, never been tested for their ability to encapsulate protein molecules, a fortiori blood coagulation proteins.

It has been shown in the examples that the incorporation of FVII/VIIa into micelles of a block copolymer as defined above does not cause any detectable modification of this coagulation factor. It is shown in the examples that the amount of FVII/VIIa that is used as the starting material for incorporation into micelles of the block copolymer described herein is integrally found in the final pharmaceutical composition which is prepared. The inventors have also shown that, starting from a given amount of FVII/VIIa for preparing (i) a pharmaceutical composition wherein FVII/VIIa is incorporated in micelles of the block copolymer described herein and (ii) a comparative composition devoid of micelles of the said block copolymer, the same amount of FVII/VIIa is measured in each of the two compositions.

Furthermore, it has been shown in the examples that, for a given amount of FVII/FVIIa incorporated respectively (i) into a composition in the absence of micelles and (ii) into a composition comprising micelles of the block copolymer defined above, an overall in vivo bioavailability of a significantly greater amount of FVII/FVIIa is observed, as illustrated by the FVII/FVIIa blood concentration profiles with each of these two compositions.

In addition, it is shown in the examples that the amount of FVII/VIIa which is released in vivo by the compositions comprising the micelles of said block copolymer into which FVII/VIIa is incorporated, even 48 hours after the time of injection, is greater than the amount of FVII/VIIa which is released in vivo by the micelle-free compositions 6 hours after the time of injection.

According to yet another aspect, it is shown in the examples that the compositions of FVII/VIIa incorporated into micelles of the block copolymer defined above are characterized in vivo in particular by an area under the curve (AUC) value which is up to two and a half times greater than the AUC value measured for the corresponding micelle-free composition.

It is noted that the AUC values of the compositions of FVII/VIIa incorporated into micelles in accordance with the invention are greater than the AUC values obtained with other known technical solutions for encapsulating FVII/VIIa, for instance that described in European patent N° EP 1 633 440 (see the results of tables 5 and 6 of said document).

According to yet another aspect, a pharmaceutical composition of the invention comprising FVII/VIIa incorporated into micelles of said copolymer allows the FVII/FVIIa to be bioavailable in the bloodstream for an exceptionally long period of time, compared with the bioavailability time of the same composition free of micelles, since mean residence times (MRTs) which are up to ten times longer have been measured with the pharmaceutical compositions of the invention.

In addition, it has been shown in the examples that the FVII/VIIa present in the pharmaceutical compositions of the invention was eliminated from the bloodstream much more slowly than the FVII/VIIa present in the corresponding pharmaceutical compositions free of micelles, since FVII/VIIa clearance (CL) values which are almost one third and lower than with the micelle-free compositions are measured after administration of the pharmaceutical compositions of the invention.

In addition, the terminal half-life values of t_{1/2}(β) for the FVII/VIIa in the bloodstream, obtained with the pharmaceutical compositions of the invention, are much greater than those observed for the corresponding compositions free of micelles.

It is added that the exceptional pharmacokinetic profiles which are obtained when the FVII/VIIa is incorporated into micelles of said block copolymer defined in the present description could not be foreseen by those skilled in the art, at least owing to the fact that the ability of micelles of this type (i) to incorporate a protein molecule, (ii) to protect it against modifications caused by its transport in the bloodstream and (iii) to release it over time in a nondegraded form and in large amounts, is disclosed for the first time in the present description.

A fortiori, those skilled in the art could not anticipate that a coagulation protein of a specific type, such as factor VII/VIIa, could be successfully incorporated into the types of micelles described herein, and then be released in vivo according to a pharmacokinetic profile compatible with the possibility of developing improved FVII/VIIa prophylactic or therapeutic treatments, in particular FVII/VIIa prophylactic or therapeutic treatments requiring a reduced number of injections for a given period of time.

The surprising and unforeseeable nature of the in vivo results obtained with the pharmaceutical compositions of FVII/FVIIa of the invention is particularly attested to by the fact that the micelles of said block copolymer as defined in the present description have proved to be completely incapable of providing a change in the pharmacokinetic profile of another coagulation factor, which has significant structural and functional similarities with FVII/VIIa, namely factor IX. It is in particular specified that factor FVII/VIIa and factor IX are both GLA-domain proteins.

More precisely, it is shown in the examples that the amounts of factor IX incorporated into the compositions comprising micelles of said block copolymer are, as for FVII/VIIa, comparable with those of factor IX incorporated into the corresponding micelle-free compositions.

However, the results of the examples also show that the profiles of change in respective plasma concentrations of factor IX incorporated into compositions comprising said micelles and into corresponding compositions free of micelles, are identical. These results mean that the micelles of said block copolymer are, with regard to factor IX, (i) either incapable of incorporating factor IX, (ii) or incapable of incorporating factor IX without degrading it, (iii) or incapable of protecting factor IX against the modifications that it may undergo in the bloodstream, (iv) or incapable of releasing the incorporated factor IX into the bloodstream, (v) or else exhibit a combination of at least two of the abovementioned inabilities.

In some embodiments of a pharmaceutical composition of the invention, said pharmaceutical composition comprises factor VII encapsulated in micelles formed from a copolymer of formula (I) below: 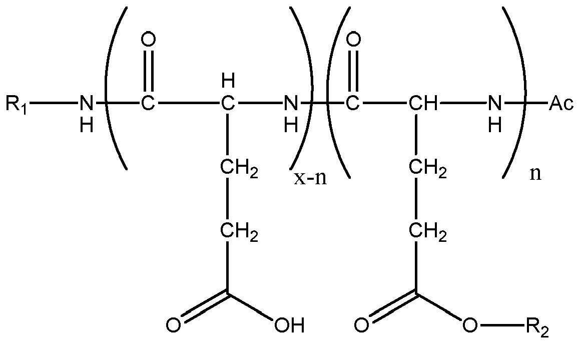 in which:
- R1 means a polyalkylene glycol chain,
- R2 is a group chosen from (i) an alkyl chain containing from 5 to 18 carbon atoms and (ii) a benzyl group,
- x is an integer ranging from 10 to 100,
- n is an integer ranging from 1 to x-1, and
- the n subunits having the hydrophobic group in the side chain and the x-n subunits having the carboxylic acid group in the side chain are randomly distributed throughout the hydrophobic polymer segment.

In the block copolymer of formula (I), "Ac" means an acetyl group.

In the block copolymer of formula (I) above, the hydrophilic segment is represented by the R1 group consisting of a polyalkylene glycol.

As used herein, polyalkylene glycol is of the following formula (A):

H-[O-R]z-OH (A),

wherein:
- R is a linear alkyl group having from 1 to 4 carbon atoms, and
- z is an integer ranging from 10 to 2500, preferably from 50 to 500. Preferably, R is an unsubstituted linear alkyl group.

In some embodiments, R consists of an ethyl group or a butyl group and z ranges from 200 to 300.

The polyalkylene glycol is advantageously chosen from a polyethylene glycol, i.e. group R of formula (A) means an ethyl group, and a polybutylene glycol, i.e. group R of formula (A) means a butyl goup.

Preferably, R1 is a polyethylene glycol.

Advantageously, R1 is a polyalkylene glycol, e.g. a polyethylene glycol or a polybutylene glycol, having a molecular mass ranging from 5kDa to 50kDa, which includes a polyalkylene glycol having a molecular mass ranging from 8kDa to 20kDa, which also includes a polyalkylene glycol having a molecular mass ranging from 10kDa to 12kDa.

Preferably, R1 is a polyethylene glycol having a molecular mass ranging from 5kDa to 50kDa, which includes a polyethylene glycol having a molecular mass ranging from 8kDa to 20kDa, which also includes a polyethylene glycol having a molecular mass ranging from 10kDa to 12kDa.

The molecular mass of a polyethylene glycol is 44m + 62, m being an integer consisting of the number of ethylene glycol units in the polymer. A polyethylene glycol of 5 kDa consequently comprises approximately 113 glycol units (i.e. z= about 113). A polyethylene glycol of 8 kDa consequently comprises approximately 180 glycol units (i.e. z= about 180). A polyethylene glycol of 10kDa consequently comprises approximately 227 glycol units. A polyethylene glycol of 12kDa consequently comprises approximately 270 glycol units. A polyethylene glycol of 15kDa consequently comprises approximately 338 glycol units. A polyethylene glycol of 20kDa consequently comprises approximately 450 glycol units. A polyethylene glycol of 50kDa consequently comprises approximately 1126 glycol units. It should be noted that the value of "x" is determined as a standard value with a numerical allowance industrially. Illustratively, for a "x" value of 40, the actual measurement value of "x" may range between 37 and 43.

The molecular masses of a polyalkylene glycol, including of a polyethylene glycol, are average molecular masses. In general, the size of a polyalkylene glycol, including of a polyethylene glycol, is defined in relation to its average molecular mass, owing to the fact that, in practice, the individual size of the polyethylene glycol molecules can vary slightly within the population of molecules of the polymer.

In the block copolymer of formula (I), the hydrophobic segment consists of a poly(glutamic acid), of which at least a part of the glutamine residues are esterified with a hydrophobic R2 group.

In some embodiments, the R2 group consists of an alkyl containing from 5 to 18 carbon atoms, which is unsubstituted or else substituted with one or more alkyls containing from 1 to 3 carbon atoms.

In some preferred embodiments, the R2 group consists of an alkyl chain containing from 8 to 12 carbon atoms.

R2 may consist of an unsubstituted alkyl chain containing 8 carbon atoms (octyl group).

In other embodiments, the R2 group consists of a benzyl group.

The hydrophobic segment of a block copolymer of formula (I) comprises "x" glutamic acid residues. x is an integer ranging from 10 to 100, which includes from 20 to 75, and which also includes from 30 to 60. The examples illustrate the use of a block copolymer of formula (I) for which x is equal to 40.

As already specified previously, a part of the amino acid residues in the hydrophobic segment is substituted with an R2 group. The hydrophobic segment may be characterized in particular by the substitution ratio for substitution with an R2 group. The substitution ratio "SR" for substitution with an R2 group is calculated according to the following formula (A): SR=n/x.

The substitution ratio SR for substitution of the hydrophobic segment with an R2 group is of at least 0.1 and of less than 1.0 generally, for example from 0.4 to less than 1.0, which includes from 0.7 to less than 1.0.

It should be noted that the substitution ratio SR for substitution with an R2 group is determined as an average value measured for a set of molecules of the corresponding block copolymer of formula (I). Thus, when the ratio SR is illustrated as 0.9, the actual measurement ratio may be between 0.79 and 0.98 due to the numerical allowance of the value "x".

In general, the block copolymers defined in the present description can be synthesized according to the methods disclosed in European patent application No. EP2042184.

The block copolymers defined in the present description spontaneously form micelles when they are present in an aqueous solution, by self-association. It is specified that the specific structure of said block copolymers confers on them the property of self-association, i.e., in particular, their ability to spontaneously form stable nanoparticles of micelle type, having a hydrophobic core and a hydrophilic crown.

Without wishing to be bound by any particular theory, the applicant believes that the FVII/VIIa which is encapsulated in the micelles of said block copolymer defined above is subsequently released into the bloodstream in two distinct ways, respectively (i) by diffusion of the FVII/VIIa molecules through the micelle and (ii) by release of the FVII/VIIa molecules when the micelles dissociate after a certain residence time in the bloodstream.

The terms "factor VII", "FVII" and "FVIIa" can be used without distinction to denote blood coagulation factor VII, as appropriate in its activated form (FVIIa), and encompass the mutants and the variants with respect to the FVII/VIIa naturally encoded in the genome of a mammal. As used herein, Factor VII encompasses FVII as well as FVIIa. The FVII/VIIa which is encapsulated in micelles in accordance with the invention is preferentially human FVII/VIIa, or a mutant or a variant of a human FVII/VIIa.

In order to prepare a pharmaceutical composition of the invention, use may be made of an FVII/VIIa purified from blood plasma or else of a recombinant FVII/VIIa, these two forms of FVII/VIIa being well known to those skilled in the art and readily commercially available.

An FVII/VIIa purified from blood plasma may, for example, be the pharmaceutical specialty marketed under the name "factor VII LFB" (French marketing authorization No. 5615752).

A recombinant human FVII/VIIa may, for example, consist of the pharmaceutical specialty marketed under the name NovoSeven® (NovoNordisk®, French marketing authorization No. 5730938 based on the European marketing authorization initially granted on 27 January 2004). In this pharmaceutical specialty, the FVII/VIIa is in a lyophilized form.

A recombinant human FVII/VIIa may also consist of an FVII/VIIa produced in rabbits which are transgenic for the gene encoding human FVII, which can be produced in accordance with the teaching of PCT application No. W02007/138199.

A recombinant FVII/VIIa may also consist of an FVII/VIIa produced in accordance with the teachings of the PCT application No. WO 2008/015339 or with the teachings of United States patent application No. US 2004185535.

As already previously specified, it also possible to use a factor VII consisting of a variant or a mutant of FVII/VIIa, in particular a variant or a mutant of human FVII/VIIa. As variants or mutants of human FVII/VIIa, use may, for example, be made of those which are described in the following documents: (i) PCT applications WO 2008127702, WO 2008155509, WO 0222776, WO 2009126307, WO 02077218, WO 03093465 and WO 0183725, and (ii) United States patents US7416860 and US7419803.

It is known in the prior art that, according to the type of FVII/VIIa under consideration, the activity of the FVII/VIIa can range from 500 to 4000 international units (IU) per milligram of FVII/VIIa protein. Illustratively, the type of FVII/FVIIa used in the examples has an activity of about 2000IU/mg FVII/FVIIa.

The examples illustrate the use of a recombinant human FVII/VIIa which has a specific activity of 2000 international units per milligram.

Advantageously, the micelles of block copolymer in which the FVII/VIIa is incorporated or encapsulated have an FVII/VIIa:copolymer mass ratio ranging from 1:100 wt:wt to 50: 100 wt:wt, better still from 5:100 wt:wt to 20: 100 wt:wt.

In general, those skilled in the art understand that the FVII/VIIa:copolymer mass ratio should have a value which is sufficiently large to allow the administration to the patient of a pharmaceutical composition comprising an amount of FVII/VIIa active ingredient that is compatible with the prophylactic or therapeutic efficacy that is desired.

Those skilled in the art also understand that the micelles of the block copolymer defined in the present description have the ability to incorporate a given amount of FVII/VIIa, with a maximum value for the FVII/VIIa:copolymer mass ratio above which the excess FVII/VIIa molecules are no longer satisfactorily incorporated into the micelles and would therefore be capable of being in free form in a pharmaceutical composition of the invention. Under conditions of a large excess mass of FVII/VIIa relative to said block copolymer, in which a part of the FVII/VIIa molecules are in free form, a modification of the pharmacokinetic profile of the corresponding FVII composition is induced, which may not comply with the objectives pursued by the present invention.

The examples illustrate pharmaceutical compositions of the invention in which the FVII/VIIa:copolymer mass ratio is 5:100 wt:wt and 10:100 wt:wt.

The pharmaceutical compositions of the invention comprising FVII/VIIa incorporated or encapsulated in micelles can be in liquid form or in lyophilized form.

The pharmaceutical compositions according to the invention may be prepared in lyophilized form. A suitable amount of sterile demineralized water is added to these lyophilized compositions in order to prepare a pharmaceutical composition of the invention in liquid form.

Those skilled in the art understand that, irrespective of the form in which a pharmaceutical composition of the invention is stored before use, mainly in solid form or in liquid form, said pharmaceutical composition is administered to the patient in liquid form.

Preferably, a pharmaceutical composition also comprises a buffer, or a combination of buffers, intended to adjust the pH value so as to stabilize the micelles in which the FVII/VIIa molecules are incorporated or encapsulated, most particularly when the pharmaceutical composition is in a liquid form.

The maintaining of the pH value of the pharmaceutical composition of the invention is in particular intended to promote the association of the FVII/VIIa molecules with the molecules of said block copolymer, within the micelles.

In some embodiments of a pharmaceutical composition of the invention, use is made of a buffer, or a combination of buffers, capable of maintaining the pH of said composition at a given pH value, in a range of pH values of from 4.5 to 6.5.

In some preferred embodiments, a buffer, or a combination of buffers, capable of maintaining the pH of said composition at a given pH value, in a range of pH values of from 5 to 6, is added.

Without wishing to be bound by any particular theory, the applicant believes that, at a given pH in the range of from pH 5 to pH 6, the interactions between the FVII/VIIa molecules and the molecules of block copolymer defined in the present description are promoted. It is specified that FVII/VIIa, and in particular a recombinant human FVII/VIIa, has an isoelectric point of between 6 and 7. In the embodiments of the pharmaceutical composition of the invention, in which the pH is maintained in a range of from pH 5 to pH 6, the molecules are in a charged form, just like the constitutive amino acid residues of the hydrophobic segment of the block copolymer, including the constitutive glutamate residues of the block copolymer of formula (I). The electrostatic interactions between the FVII/VIIa molecules and the molecules of said block copolymer are, in this pH range of from 5 to 6, overall attractive.

The examples illustrate embodiments of a pharmaceutical composition of the invention comprising a buffer capable of maintaining the pH of said composition in liquid form at a pH of 5, or else at a pH of 6.

Any type of physiologically acceptable buffer known to those skilled in the art can be used. A buffer may be present in a pharmaceutical composition of the invention in a final concentration range of from approximately 2mM to 50mM.

Use may in particular be made of a buffer, or a combination of buffers, chosen from organic acids or organic acids or salts thereof, such as:
- citrate buffers, including monosodium citrate or disodium citrate, or a mixture thereof, a mixture of citric acid and of trisodium citrate, a mixture of citric acid and of trisodium citrate,
- succinate buffers, including a mixture of succinic acid and of monosodium succinate, a mixture of succinic acid and of sodium hydroxide, a mixture of succinic acid and of monosodium succinate,
- tartrate buffers, including a mixture of tartaric acid and of sodium tartrate, a mixture of tartaric acid and of potassium tartrate, a mixture of tartaric acid and of sodium hydroxide,
- fumarate buffers, including a mixture of fumaric acid and of monosodium fumarate, a mixture of fumaric acid and of disodium fumarate, a mixture of monosodium fumarate and of disodium fumarate,
- gluconate buffers, including a mixture of gluconic acid and of sodium gluconate, a mixture of gluconic acid and of sodium hydroxide, a mixture of gluconic acid and of potassium gluconate,
- oxalate buffers, including a mixture of oxalic acid and of sodium oxalate, a mixture of oxalic acid and of sodium hydroxide, a mixture of oxalic acid and of potassium oxalate,
- lactate buffers, including a mixture of lactic acid and of sodium lactate, a mixture of lactic acid and of sodium hydroxide, a mixture of lactic acid and of potassium lactate,
- acetate buffers, including a mixture of acetic acid and of sodium acetate, a mixture of acetic acid and of sodium hydroxide,
- phosphate buffers, including a mixture of disodium chlorophosphate and of monopotassium phosphate, a mixture of dibasic monohydrogen phosphate and of monobasic dihydrogen phosphate.

The examples illustrate pharmaceutical compositions of the invention comprising a disambiguation (2-(N-morpholino) ethanesulfonic acid or MES) buffer. The MES buffer can be used at a final concentration of 10mM.

Furthermore, a pharmaceutical composition of the invention comprising FVII/VIIa encapsulated in micelles formed from the block copolymer defined in the present description may also comprise a cryoprotective agent or a combination of cryoprotective agents.

The cryoprotective agent may be any type of cryoprotective agent well known to those skilled in the art. Use may in particular be made of a cryoprotective agent of the sugar type, for example a monosaccharide, a disaccharide or a polysaccharide. Use may, for example, be made of a cryoprotective agent chosen from DMSO, ethylene glycol, glycerol, sucrose or trehalose.

The examples illustrate pharmaceutical compositions of the invention in which the cryoprotective agent is sucrose.

A pharmaceutical composition according to the invention may comprise one or more physiologically acceptable additional excipients among those which are recommended by the European pharmacopeia or the US pharmacopeia in force. A pharmaceutical composition of the invention may comprise one or more additional excipients recommended in the 6th edition of the European Pharmacopeia of June 2007 or of the US Pharmacopeia of 2010.

When a pharmaceutical composition of the invention is in liquid form, in particular when it is in the form of a ready-to-use liquid, said pharmaceutical composition comprises a final concentration of said block copolymer defined in the present description in the form of micelles ranging from 1mg/ml to 100mg/ml.

Without wishing to be bound by any particular theory, the applicant believes that, at a lower final concentration of said block copolymer, the integrity of the micelles may be modified owing to the fact that the critical minimum concentration (CMC) for maintaining the arrangement of the molecules of said block copolymer in the micelle form is not reached.

At a concentration of said block polymer of greater than 100mg/ml, the applicant believes that the viscosity characteristics of said pharmaceutical composition would lead to a reduced compliance of the said composition with its administration to the patient by injection.

In some preferred embodiments, a pharmaceutical composition in accordance with the invention, when it is in liquid form, in particular when it is in a ready-to-use liquid form, has a final concentration of said block copolymer, for example of the block copolymer of formula (I), ranging from 1mg/ml to 50mg/ml, including ranging from 5 mg/ml to 30mg/ml.

In general, a pharmaceutical composition of the invention comprises an amount of FVII/VIIa suitable for obtaining activity of FVII/VIIa in a range of from 500 IU to 10 000 IU per dosage unit.

By way of illustration, for an FVII/VIIa having a specific activity of 2000 IU/mg, a dosage unit of the pharmaceutical composition of the invention at 5000 IU comprises 2.5mg of FVII/FVIIa incorporated or encapsulated in micelles. Furthermore, for a pharmaceutical composition of the invention in which the FVII/VIIa:copolymer mass ratio is 10:100, said dosage unit comprises an amount of said copolymer in the form of micelles, for example of the copolymer of formula (I), which is 25mg of said copolymer. Finally, in one embodiment of the pharmaceutical composition in which the final concentration of said copolymer is 25mg/ml, said dosage unit, when it is in a ready-to-use liquid form, has a volume of 1ml.

In some embodiments of a pharmaceutical composition according to the invention wherein the said composition consists of a liquid solution, the said composition comprises from 0.1mg/ml to 10mg/ml of FVII/FVIIa. In some of these embodiments, the said composition comprises from 0.5mg/ml to 5mg/ml of FVII/FVIIa. These embodiments encompass pharmaceutical compositions comprising about 1mg/ml FVII/FVIIa.

It is understood that, in some presentation forms of a pharmaceutical composition of the invention, said composition in liquid form can be packaged in a bottle containing a volume sufficient for a plurality of dosage units, which are removed in order to successively fill the reservoir of the syringes which are subsequently used for the administration of said composition, by parenteral injection.

Generally, a pharmaceutical composition of the invention is suitable for parenteral administration thereof, which encompasses subcutaneous, intradermal and intravenous administration. Intravenous administration is preferred.

A pharmaceutical composition of the invention is generally intended for the prevention or treatment of coagulation disorders. A pharmaceutical composition according to the invention is used in any of the known medical indications and in any of the medical indications that will subsequently be identified for FVII/VIIa in general.

A pharmaceutical composition of the invention may, for example, be used for the treatment of haemorrhagic events related to an isolated constitutional deficiency in factor VII.

A pharmaceutical composition of the invention may also be used for the prevention of haemorrhagic events in the case of an isolated constitutional deficiency in factor VII and associated with a history in the patient.

A pharmaceutical composition according to the invention is in particular indicated in the treatment of haemorrhagic episodes and in the prevention of haemorrhage occurring during the surgical procedures or invasive procedures for the following groups of patients:
- in patients who have congenital haemophilia with inhibitors directed against coagulation factors VIII having a titre of > 5 Bethesda units (BU),
- in patients with congenital haemophilia in whom a strong anamnestic response to the administration of factor VIII is foreseeable.

A pharmaceutical composition according to the invention is in particular indicated in the treatment of haemorrhagic episodes and in the prevention of haemorrhage occurring during surgical procedures or invasive procedures for the following groups of patients:
- in patients who have congenital haemophilia with inhibitors directed against coagulation factors IX having a titre of > 5 Bethesda units (BU),
- in patients with congenital haemophilia in whom a strong anamnestic response to the administration of factor IX is foreseeable,
- in patients with acquired haemophilia,
- in patients with a congenital FVII deficiency, and
- in patients with Glanzmann's thrombasthenia with anti-GP IIb-IIIa and/or anti-HLA antibodies, and who show a lack of response (past or present) to platelet transfusions.

According to yet another aspect, the present invention relates to a pharmaceutical composition comprising factor VII encapsulated in micelles formed from a block copolymer molecules containing a hydrophilic polymer segment and a hydrophobic polymer segment consisting of a polyamino acid, which composition, when it is administered parenterally, is suitable for generating a pharmacokinetic profile having one or more of the following characteristics:
- an AUC (area under the curve) value which is of 10% or more higher than the AUC value obtained with a comparative composition wherein the same amount of FVII is not encapsulated,
- an MRT (mean residence time) value of at least 2 hours,
   and
- a CL (clearance) value of at most 200ml/kg/h.

As mentioned above, the values of the MRT and CL should be obtained from seven-week-old rats of the Wistar strain.

Embodiments of a pharmaceutical composition above are described in detail throughout the whole present description.

The invention also relates to a factor VII encapsulated in micelles formed from block copolymer molecules containing (i) a hydrophilic polymer segment consisting of a polyalkylene glycol and of (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for use thereof as a medicament.

The invention also relates to a factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for the treatment of coagulation disorders.

The invention also relates to the use of a factor VII encapsulated in micelles formed from a block copolymer molecule containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for producing a medicament for the treatment of coagulation disorders.

By way of illustration, a pharmaceutical composition of the invention may be used for the treatment of haemorrhagic events, for example:
- by administration of an amount of said composition necessary for a dose of from 15 to 20IU/kg of FVII/VIIa in moderate events, and
- by administration of an amount of said composition necessary for a dose of from 30 to 40IU/kg of FVII/VIIa in serious events, followed by a dose of 20IU/kg of FVII/VIIa at successive intervals of 6 to 8 hours, in order to maintain a factor VII level of between 35% and 45% until resolution of the haemorrhagic event.

By way of illustration, a pharmaceutical composition of the invention may be used for treatment in the context of a surgical procedure: in order to obtain a circulating factor VII level of greater than 50% at the time of the surgical procedure, an amount of pharmaceutical composition according to the invention necessary for a loading dose of from 30 to 40IU/kg of FVII/VIIa is injected one hour before the surgical act and the factor VII level is tested immediately before the procedure. The subsequent doses of the pharmaceutical composition of the invention will be administered every 6 to 8 hours, so as to maintain over the next few days:
- in the case of a minor surgical procedure, a circulating factor VII level of 20 to 30%;
- in the case of a maj or surgical procedure, a circulating factor VII level of 3 5 to 45%.

The duration of the factor VII level correction and the biological monitoring are suitable for the clinical situation and the risk of haemorrhage.

By way of illustration, a pharmaceutical composition of the invention may be used for a prophylactic treatment: the pharmaceutical composition according to the invention may be indicated in the prevention of repeated haemorrhagic events, for example haemarthrosis, in individuals with a factor VII deficiency of < 5%. The useful dose of the pharmaceutical composition of the invention is that necessary for a dosage of from 20 to 30 IU/kg of FVII/VIIa and should be injected at least two to three times a week for several weeks to several months. It should be modulated according to the results regarding the prevention of bleeding.

The present invention is also illustrated in a nonlimiting manner by the following examples:

### EXAMPLES

### Example 1: First series of attempts to prepare a pharmaceutical composition comprising FVII/VIIa encapsulated in micelles of block copolymer of formula (I)

### A. Method for preparing the pharmaceutical composition

### A.1. Copolymer

Use was made of two types of self-associating copolymers of formula (I) of which the R₂ group is (i) either an unsubstituted octyl chain, denoted "PEG-pGlu(OC8)-Ac", (ii) or a benzyl group, denoted "PEG-pGlu(OBn)-Ac".

The PEG-pGlu(OC8)-Ac copolymer has the following formula (I-A): 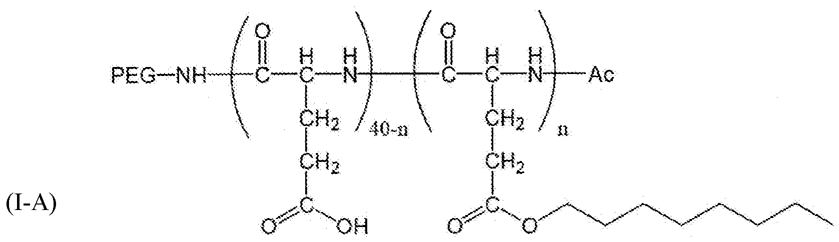 in which: the R₁ group is a polyethylene glycol chain having a molecular mass of 10kDa, and the poly(glutamate) chain of which comprises 40 glutamate units (x=40) wherein the substitution ratio for substitution of the glutamate residues with the octyl group is 0.9 (36 glutamate units are substituted out of the 40 constituent glutamate units of the poly(glutamate) chain).

The PEG-pGlu(OBn)-Ac copolymer has the following formula (I-B): 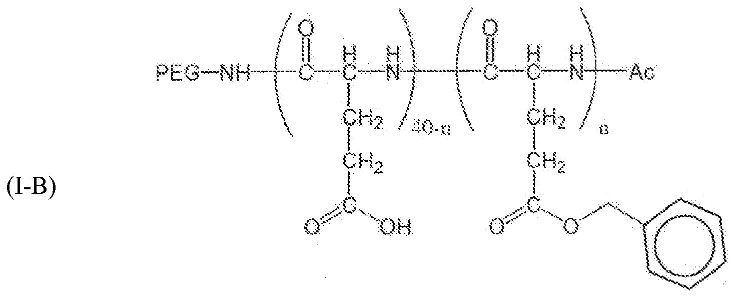 in which: the R₁ group is a polyethylene glycol chain having a molecular mass of 10kDa, of which the poly(glutamate) chain comprises 40 glutamate units (x=40)wherein the substitution ratio for substitution of the glutamate residues with the benzyl group is 0.9 (36 glutamate units are substituted out of the 40 constituent glutamate units of the poly(glutamate) chain).

### A.2. Factor VII/VIIa

Use was made of recombinant human factor VII/VIIa produced in rabbits' milk in accordance with the teaching of PCT application No. WO 2007/138199 in the name of the Laboratoire Français du Fractionnement et des Biotechnologies.

The purified recombinant human FVII/VIIa has a specific activity of 2000IU/mg.

### A.3. Preparation of the pharmaceutical composition comprising FVII/VIIa encapsulated in micelles of the PEG-pGlu(OC8)-Ac copolymer or in micelles of the PEG-pGlu(OBn)-Ac copolymer

### A.3.1. Composition comprising micelles of PEG-pGlu(OC8)-Ac

The following are added to a final volume of 2.4ml of an aqueous solution containing a 10mM MES buffer at pH 5 and 10% sucrose: (i) 0.36mg of the recombinant human FVII/VIIa and (ii) 3.6mg of the PEG-pGlu(OC8)-Ac, at 4°C.

In the mixture, the FVII/VIIa and the PEG-pGlu(OC8)-Ac are in a mass ratio of 10:100.

After a period of incubation of the above mixture of 24 hours at 4°C, the PEG-pGlu(OC8)-Ac molecules, which are self-associating, formed micelles encapsulating the recombinant human FVII/VIIa.

### A.3.2. Composition comprising micelles of PEG-pGlu(OBn)-Ac

The following are added to a final volume of 2.4ml of an aqueous solution containing a 10 mM MES buffer at pH5: (i) 0.36mg of the recombinant human FVII/VIIa and (ii) 3.6mg of the PEG-pGlu(OBn)-Ac, at 4°C.

In the mixture, the FVII/VIIa and the PEG-pGlu(OBn)-Ac are in a mass ratio of 10:100.

After a period of incubation of the above mixture of 24 hours at 4°C, the PEG-pGlu(OBn)-Ac molecules, which are self-associating, formed micelles encapsulating the recombinant human FVII/VIIa.

### B. Pharmacokinetic assays

### B.1. Animals

Seven-week-old rats of the Wistar strain were used.

### B.2. Administration protocol

Groups of three rats were used, the rats of the same group receiving the same treatment.

A micelle-free FVII/VIIa solution was administered i.v. at a dose of 600 IU/kg to a first group of three rats.

A solution of FVII/VIIa encapsulated in micelles of PEG-pGlu(OC8)-Ac was administered i.v. at a dose of 600 IU/kg to a second group of three rats.

A solution of FVII/VIIa encapsulated in the micelles of PEG-pGlu(OBn)-Ac was administered i.v. in the tail, at a dose of 600 IU/kg, to a third group of three rats.

### B.3. Measurement of pharmacokinetic profiles

The pharmacokinetic profiles for each of the three formulations were produced with the FVII ELISA detection kit sold by the company Diagnostica Stago under the name "Asserachrom^{®} VII:Ag", using the following protocol:
- rat plasma are pre-treated for 1h at 4°C with a surfactant solution at a final concentration of 0.1% to disolve the micelles and release all the FVIIa still entrapped in the micelle.
- the standard curve is adapted in the presence of micelles to take into account the potential interference of known amount of polymer (Figue 1).

The plasma FVII/VIIa concentration was measured for each of the rats at the following times after injection: 5 minutes, 0.5 hour, 1 hour, 2 hours, 8 hours, 24 hours and 48 hours.

The results are illustrated in Figure 2.

The results in Figure 2 show that the FVII/VIIa encapsulated in micelles of PEG-pGlu(OC8)-Ac or of PEG-pGlu(OBn)-Ac is continually released over time with a significant amount of FVII/VIIa still being released after 24 hours (PEG-pGlu(OBn)-Ac) and even after 48 hours (PEG-pGlu(OBn)-Ac).

### Example 2: Second series of attempts to prepare a pharmaceutical composition comprising FVII/VIIa encapsulated in micelles of block copolymer of formula (I)

### A. Method for preparing the pharmaceutical composition

The protocol described in example 1 was used to prepare the following five formulations:
- a formulation of FVII/VIIa free of micelles in a MES buffer solution at pH 5 that may be referred to as "FVII solution (pH 5)" hereinafter;
- a formulation of FVII/VIIa free of micelles in a phosphate buffer solution at pH 6 that may be referred to as "FVII solution (pH 6)" hereinafter;
- a formulation of FVII/VIIa encapsulated in micelles of PEG-pGlu(OC8)-Ac, in an FVII/:PEG-pGlu(OC8)-Ac mass ratio of 0.5:10 at pH 5 that may be referred to as "5% C8 Micelle (pH 5)" hereinafter;
- a formulation of FVII/VIIa encapsulated in micelles of PEG-pGlu(OC8)-Ac, in an FVII/:PEG-pGlu(OC8)-Ac mass ratio of 1:10 at pH 5 that may be referred to as "10% C8 Micelle (pH 5)" hereinafter; and
- a formulation of FVII/VIIa encapsulated in micelles of PEG-pGlu(OBn)-Ac, in an FVII/:PEG-pGlu(OBn)-Ac mass ratio of 1:10 at pH 5 that may be referred to as "10% Bn Micelle (pH 5)" hereinafter.

### B. Pharmacokinetic assays

The pharmacokinetic assays were carried out under the same conditions as in example 1.

The results are illustrated in Figure 3.

The results in Figure 3 show that the FVII/VIIa encapsulated in micelles of PEG-pGlu(OC8)-Ac or of PEG-pGlu(OBn)-Ac is continually released over time with a significant amount of FVII/VIIa still being released after 24 hours (PEG-pGlu(OBn)-Ac and even after 48 hours (PEG-pGlu(OBn)-Ac).

The results in Figure 3 also show that an identical pharmacokinetic profile is obtained with the two formulations of FVII/VIIa encapsulated, respectively, (i) in 5% C8 Micelle (pH 5) and (ii) in 10% C8 Micelle (pH 5).

The various characteristic values of the pharmacokinetic profiles of some of the formulations tested are reported in table 1 below:

**Table I**

| **Parameter** | | **FVII Solution (pH 6)** | **10% C8 Micelle (pH 5)** | C8/Solution ratio | **10% Bn Micelle (pH 5)** | Bn/Solution ratio |
|---|---|---|---|---|---|---|
| AUC | (mIU/ml h) | **1734** | **5052** | 29 | **3981** | 23 |
| t_{1/2}(α) | (h) | **0.18** | **0.78** | | **0.74** | |
| t_{1/2}(β) | (h) | **1.06** | **11.65** | 110 | **4.61** | 44 |
| MRT | (h) | **0.69** | **8.23** | 119 | **2.54** | 37 |
| CL | (ml/kg/h) | **346** | **119** | | **151** | |

The results in Table I illustrate the best pharmacokinetic profile parameters obtained with the various embodiments of a pharmaceutical composition comprising recombinant human FVII/VIIa encapsulated in micelles formed from a copolymer of formula (I).

The value of the AUC of the compositions of FVII/VIIa encapsulated in micelles of a copolymer of formula (I) may in particular be noted, said value being, respectively, 2.3 times greater (PEG-pGlu(OBn)-Ac) and 2.9 times greater (PEG-pGlu(OC8)-Ac) than the AUC measured for the corresponding formulation of FVII/VIIa which is free of micelles.

In addition, the results in table I show that the MRT residence time value for the FVII/VIIa in the bloodstream is, respectively, 3.7 times greater (PEG-pGlu(OBn)-Ac) and 11.9 times greater (PEG-pGlu(OC8)-Ac) than the MRT value measured for the corresponding formulation of FVII/VIIa which is free of micelles.

Finally, the results in table 1 show that the pharmacokinetic profile obtained with the PEG-pGlu(OC8)-Ac-based formulation is more advantageous than the pharmacokinetic profile obtained with the PEG-pGlu(OBn)-Ac-based formulation.

### Example 3: Third series of attempts to prepare a pharmaceutical composition comprising FVII/VIIa encapsulated in micelles of block copolymer of formula (I)

### A. Method for preparing the pharmaceutical composition

The protocol described in example 1 was used to prepare the following three formulations:
- a formulation of FVII/VIIa free of micelles in a MES buffer solution at pH 5;
- a formulation of FVII/VIIa encapsulated in micelles of PEG-pGlu(OC8)-Ac, in an FVII/:PEG-pGlu(OC8)-Ac mass ratio of 0.5:10 at pH 5;
- a formulation of FVII/VIIa encapsulated in micelles of PEG-pGlu(OBn)-Ac, in an FVII/:PEG-pGlu(OBn)-Ac mass ratio of 0.5:10 at pH 5 that may be referred to as "5% Bn Micelle (pH 5)" hereinafter.

### B. Pharmacokinetic assays

The pharmacokinetic assays were carried out under the same conditions as in example 1.

The results are illustrated in Figure 3.

The results in Figure 3 show that the FVII/VIIa encapsulated in micelles of PEG-pGlu(OC8)-Ac or of PEG-pGlu(OBn)-Ac is continually released over time with a significant amount of FVII/VIIa still being released after 24 hours (PEG-pGlu(OBn)-Ac and even after 48 hours (PEG-pGlu(OBn)-Ac).

The characteristic values of the pharmacokinetic profiles of the three formulations tested are reported in table II below:

**Table II**

| **Parameter** | | **FVII Solution (pH 5)** | **5% C8 Micelle (pH 5)** | C8/Solution ratio | **5% Bn Micelle (pH 5)** | Bn/Solution ratio |
|---|---|---|---|---|---|---|
| AUC | (mIU/ml h) | **1260** | **5014** | 40 | **4073** | 32 |
| t_{1/2}(α) | (h) | **0.20** | **0.74** | | **0.81** | |
| t_{1/2}(β) | (h) | **1.28** | **10.25** | 80 | **4.32** | 34 |
| MRT | (h) | **0.86** | **6.86** | 79 | **2.62** | 30 |
| CL | (ml/kg/h) | **476** | **120** | | **147** | |

The results in table II illustrate the better pharmacokinetic profile parameters obtained with the various embodiments of a pharmaceutical composition comprising recombinant human FVII/VIIa encapsulated in micelles formed from a copolymer of formula (I).

The value of the AUC of the compositions of FVII/VIIa encapsulated in micelles of a copolymer of formula (I) may in particular be noted, said value being, respectively, 3.2 times greater (PEG-pGlu(OBn)-Ac) and 4.0 times greater (PEG-pGlu(OC8)-Ac) than the AUC measured for the corresponding formulation of FVII/VIIa which is free of micelles.

In addition, the results in table II show that the MRT residence time value for the FVII/VIIa in the bloodstream is, respectively, 3.0 times greater (PEG-pGlu(OBn)-Ac) and 8 times greater (PEG-pGlu(OC8)-Ac) than the MRT value measured for the corresponding formulation of FVII/VIIa which is free of micelles.

Finally, the results in table II show that the pharmacokinetic profile obtained with the PEG-pGlu(OC8)-Ac-based formulation is more advantageous than the pharmacokinetic profile obtained with the PEG-pGlu(OBn)-Ac-based formulation.

Furthermore, Figure 4 illustrates a summary of the pharmacokinetic results obtained for the various formulations tested in examples 2 and 4.

The results in Figure 4 show that, generally, superior pharmacokinetic profiles are obtained irrespective of the composition comprising FVII/VIIa encapsulated in micelles of copolymer of formula (I) that was tested.

The results in Figure 5 also show that the AUC values of the FVII/VIIa are similarly increased, regardless of the FVII:copolymer ratio in the range described in the examples, i.e. from 5:100 to 10: 100..

For all the formulations comprising micelles that were tested, a considerable increase in the mean residence time (MRT) of the FVII/VIIa in the bloodstream was measured.

The results in Figure 4 also show that better results are obtained with the formulations comprising the FVII/VIIa encapsulated in micelles formed from PEG-pGlu(OC8)-Ac than with the formulations comprising the FVII/VIIa encapsulated in micelles formed from PEG-pGlu(OBn)-Ac.

### EXAMPLE 4: Comparative example of a formulation of FIX encapsulated in micelles formed from PEG-pGlu(OC8)-Ac or from PEG-pGlu(OBn)-Ac

### A. Method for preparing the comparative formulations

The protocol described in example 1 was used to prepare the following four formulations:
- a formulation of FIX free of micelles in a phosphate buffer solution at pH 4 that may be referred to as "FIX solution (pH 4)" hereinafter;
- a formulation of FIX free of micelles in a phosphate buffer solution at pH 6 that may be referred to as "FIX solution (pH 6)" hereinafter;
- a formulation of FIX encapsulated in micelles of PEG-pGlu(OC8)-Ac, in an FIX:PEG-pGlu(OC8)-Ac mass ratio of 1:10, in a phosphate buffer solution at pH 4 that may be referred to as "10% C8 Micelle (pH 4)" hereinafter;
- a formulation of FIX encapsulated in micelles of PEG-pGlu(OC8)-Ac, in an FIX:PEG-pGlu(OC8)-Ac mass ratio of 1:10, in a phosphate buffer solution at pH 6 that may be reffered to as "10% C8 Micelle (pH 6)" hereinafter;
- a formulation of FIX encapsulated in micelles of PEG-pGlu(OBn)-Ac, in an FIX:PEG-pGlu(OBn)-Ac mass ratio of 1:10, in a phosphate buffer solution at pH 4 that may be reffered to as "10% Bn Micelle (pH 4)" hereinafter; and
- a formulation of FIXencapsulated in micelles of PEG-pGlu(OBn)-Ac, in an FIX:PEG-pGlu(OBn)-Ac mass ratio of 1:10, in a phosphate buffer solution at pH 6 that may be reffered to as "10% Bn Micelle (pH 6)" hereinafter.

Purified human FIX sold by LFB under the name Betafact^{®} was used for producing the formulations.

### C. Pharmacokinetic assays

The pharmacokinetic assays were carried out under the same conditions as in example 1, using FIX at a dose of 200 IU/kg, using the FIX ELISA detection kit sold by the company Diagnostica Stago under the name "Asserachrom^{®} IX:Ag"

The results are illustrated in Figure 6.

The results in Figure 7 show that the FIX encapsulated in micelles of PEG-pGlu(OC8)-Ac or of PEG-pGlu(OBn)-Ac is released according to kinetics identical to those of the FIX present in the formulations free of micelles.

Thus, the results in Figure 6 illustrate that the formulation of human FIX in micelles formed from a copolymer of formula (I) does not modify its pharmacokinetic profile.

These results show that the technical solution which was developed according to the invention for preparing improved pharmaceutical compositions of FVII/VIIa is not at all transposable to the preparation of improved compositions of another coagulation factor, which nevertheless has many structural and functional characteristics in common with FVII/VIIa.

### Example 5: Preservation of FVII activity after FVII encapsulation in block copolymer micelles

### A. Materials and Methods

### A.1. Products

The objectives of Example 5 was to test the preservation of factor VII activity in a pharmaceutical composition comprising factor VII encapsulated in micelles of a block-copolymer of formula (I) described in the present specification.

In this example, the said pharmaceutical composition is also termed FVIIa-SR (for FVIIa sustained release).

The test was performed by including a comparative pharmaceutical composition comprising a non-encapsulated FVII factor. In this example, the said comparative pharmaceutical composition is also termed FVIIa-IR (for FVIIa Immediate Release).

The source of FVIIa was a recombinant human FVIIa purified from the milk of rabbits transgenic for human FVII in accordance with the teaching of PCT application No. WO 2007/138199 in the name of Laboratoire Français du Fractionnement et des Biotechnologies.

In the FVIIa-SR pharmaceutical composition, FVIIa was encapsulated in micelles of pGlu(OC8)-Ac with a protein/polymer mass ratio of 10:100 in an aqueous solution containing sodium at citrate 1.5g/L and 10% sucrose. The pH was adjusted to 5.2. The FVIIa concentration was 0.4g/L to 1.1g/L.

### A.2. Elisa assay

FVIIa titration is performed with the FVII ELISA kit Asserachrom® VII: Ag from Diagnostica Stago, according to the instructions of use of the manufacturer.

### A.2. FVII clotting time test

FVIIa titration by the chronometric method performed on the BCS XP machine (Siemens) consists in a measurement of the clotting time in presence of recombinant soluble tissue factor, phospholipids and calcium, in a solution where all the factors are in excess except for FVIIa, FVIIa being brought by the dilution of the sample. The FVIIa titration was performed according to the instruction of use of the manufacturer.

Both for the ELISA and for the FVII clotting time test, samples are pre-treated for 1 hour at 4°C with a surfactant solution (NP-40 at a final concentration of 0.1%) to dissolve the micelles and release all the FVIIa previously entrapped into the micelles.

### B. Results

The results are presented in the Table III below.

**Table III**

| | **nominal concentration** | **FVII Ag (IU/mL)** | **FVIIa (IU/mL)** | **FVIIa/FVIIAg** |
|---|---|---|---|---|
| FVIIa-IR | 0.4g/L | 838 | 15797 | 18.8 |
| FVIIa-IR | 0.8g/L | 1679 | 34852 | 20.8 |
| FVIIa-SR | 0.4g/L | 906 | 15406 | 17 |
| FVIIa-SR | 1.1g/L | 2214 | 42935 | 19.4 |

The results presented in Table III above show that the FVIIa activity is not altered when FVIIa is encapsulated in the micelles of block copolymer described in this patent application.

### Example 6: Study of efficacy of a pharmaceutical composition comprising factor VII encapsulated in micelles of a block co-polymer

### A. Objectives

The objectives of Example 6 was to test the *in vivo* efficacy of a pharmaceutical composition comprising factor VII encapsulated in micelles of a block-copolymer of formula (I) described in the present specification.

In this example, the said pharmaceutical composition is also termed FVIIa-SR (for FVIIa sustained release).

The test was performed by including a comparative pharmaceutical composition comprising a non-encapsulated FVII factor. In this example, the said comparative pharmaceutical composition is also termed FVIIa-IR (for FVIIa Immediate Release).

A tail-clip model was used as efficacy test. In this model, the tail extremity of factor VIII deficient mice or Hemophilia A mice is amputated. These mice have less than 1% of normal factor VIII activity and have increased blood loss in the tail-clip model compared to wild-type mice of the same strain.

The amount of blood loss was measured to compare the homeostatic effect of FVIIa-SR and FVIIa-IR. Two doses of FVIIa-SR and FVIIa-IR (4mg/kg and 8mg/kg) were administered and two time points after injection (5 minutes and 120 minutes) were selected to measure blood loss. FVIIa-SR and FVIIa-IR efficacies were evaluated using negative and positive controls, i.e. saline (NaCl 0.9%) and factor VIII-injected hemophilia A mice, respectively.

### B. Materials and Methods

### B.1. Products

### B.1.1. Active ingredients

The source of FVIIa was a recombinant human FVIIa purified from the milk of rabbits transgenic for human FVII in accordance with the teaching of PCT application N° WO 2007/138199 in the name of Laboratoire Français du Fractionnement et des Biotechnologies.

In the FVIIa-SR pharmaceutical composition, FVIIa was encapsulated in micelles of pGlu(OC8)-Ac with a protein/polymer mass ratio of 10:100 in an aqueous solution containing sodium at citrate 1.5g/L and 10% sucrose. The pH was adjusted to 5.2. The FVIIa concentration was 0.4g/L to 1.1g/L

Batch samples of FVIIa-SR were prepared at a final protein concentration of 0.4g/L.

Batch samples of FVIIa-IR were prepared at a final protein concentration of 0.4g/L.

All batch samples were stored at -70°C until use. For the test, samples were thawed at room temperature and injected to animals within three hours.

### B.1.2. Positive controls

Commercial human plasma-derived factor VIII Factane ® (FAC, LFB, Les Ulis, France) at 1000 IU/mL was used as factor VIII. Each vial was kept at 4°C until use. FVIII was resuspended to 50 IU/mL with 20mL distilled water, then diluted to 10 IU/mL in PBS (PAA laboratories, Les Mureaux, France) and injected to animals within three hours.

### B.1.3. Negative control product

Sodium chloride solution at 0.9% was used as negative control product in hemophilia A. This product was stored at room temperature.

### B.2. Animals

### B.2.1. Genetic background and housing

A factor VIII deficient mice colony was used. 85 mice of 8 to 12 weeks old were used. Breeding pairs of exon 16 factor VIII-deficient mice or hemophilia A mice were purchased from The Jackson laboratory (Bar Harbor, ME, USA). These animals were backcrossed to C57BL/6 WT mice (Janvier, Le Genest-St-Isle, France) for 8 generations. Control C57BL/6 WT mice have been derived from the last backcrosses of hemophilia A mice to ensure similar genetic background. Mice were housed as recommended by French regulations and the experimental guidelines of the European Union. Temperature, hygrometry, ventilation were controlled and recorded. A dried standard rodent diet as well as a fresh water were offered *ad libitum.*

### B.2.2. Treatments

A total of 85 hemophilia A mice were used in this study. At the time of the experiment, mice were weighed for anaesthetics and products dosage. Tails were immersed in a water-bath at 37°C 3 minutes before product administration. Then, mice were placed in a mouse restrainer to adequately restrain the animal while having access to its tail vein for products administration. Immediately after product administration, mice were anesthetized by intraperitoneal injection of tribromoethanol at a dosage of 0.02mL/g body weight of a 1.25% solution. During the experiment the temperature of the animal was kept constant to 37°C.

### B.2.3. Administration schedule

The tested products were injected as disclosed in Table IV hereunder. Briefly, two doses of FVIIa-IR or FVIIa-SR were administered to hemophilia A mice. The first dose was of 4mg/kg for each product injected to 10 mice. As control, FVIII at a dose of FVIII:C of 100IU/kg was injected to 11 hemophilia A mice. Furthermore, 19 hemophilia A mice were injected with NaCl 0.9%. For all products, the volume injected was of 200 to 250 µl depending of the dose and the concentration of the product injected. Based on the results obtained for this dose of FVIIa(SR (4mg/kg), another dose of 8mg/kg was tested. The same number of animals (10) was included for this new dose. The effect of these doses was evaluated in the tail-clip model 5 minutes after product administration (See details in Table IV below). Then, 15 and 18 hemophilia A mice were injected with 8mg/kg of FVIIa-IR and FVIIa-SR, respectively. The effect of these doses was evaluated in the tail-clip model 120 minutes after product administration.

**Table IV: Administration schedule**

| **Groups** | **Products** | **Dose protein*** | **Number of animals** | **Time of tail transaction after product administration** |
|---|---|---|---|---|
| I | NaCl 0.9% | 0 | 19 | 5 min |
| II | FVIII | 100 IU/kg | 11 | 5 min |
| III | FVIIa-IR | 4mg/kg | 10 | 5 min |
| IV | FVIIa-SR | 4mg/kg | 10 | 5 min |
| V | FVIIa-SR | 8mg/kg | 10 | 5 min |
| VI | FVIIa-IR | 8mg/kg | 15 | 120 min |
| VII | FVIIa-SR | 8mg/kg | 18 | 120 min |

| | | | | |
|---|---|---|---|---|
| * Volume injected between 200 and 250µl depending of the concentration of the product used and of the weight of hemophilia A mice. | | | | |

### B.2.4. Tail-clip model

### Tail-clip or transaction tail model

Mouse tail-bleeding time was performed using the tail-clip assay under tribromoethanol anesthesia. A scalpel was used to cut 3 mm of the tail-tip of anesthesized mice. In a first group of animals, to determine the optimal dose of FVIIa-SR efficiency, the transection of the tail was 5 minutes after the product administration. Then, in a second group of animals, the transaction was made at a time point when FVIIa-IR efficiency cannot be detected (2 hours) while hopefully FVIIa-SR still could be efficient to correct bleeding loss of hemophilia A mice.

### Parameters studied

The amputated tail was immersed immediately after transaction in a 50ml tube full of warm physiological saline. Blood was collected for 30 min at 37°C. After 30 min, the mixture of blood and physiological saline was centrifuged at 1500g. The red blood cells pellet was then be lysed in H₂O and the amount of haemoglobin was obtained by reading the absorbance at 416 nm. The volume of blood lost in each sample was calculated from a standard curve, which was obtained by lysing defined volume (20µl, 40µl, 60µL, 80µl and 100µl) of mouse blood in H₂O to extract haemoglobin as described above. At the end of the experiment all animals were anesthetized and euthanized by cervical dislocation.

### B.2.5. Statistical analysis

The Mann-Whitney U-test determined statistical differences in blood loss between two groups using the KaleidaGraph® software (version 1.0, Synergy Software, Reading, PA, USA). Stastistical significance was accepted when P<=0.05.

### C. Results

### C.1. Test controls of FVIII and NaCl 0.9% in tail-clip model 5 minutes after injection

To validate the tail-clip model in hemophilia A mice, FVIII was tested at 100IU/kg. Blood loss was measured 5 min after FVIII or NaCl 0.9% administration. Results are presented in Table V and Figure 7.
Administration of FVIII at 100 IU/kg induced a significant decrease in blood loss (P=0.0001, Mann-Whitney U-test) compared with the negative control group (NaCl 0.9%).

**Table V: Blood loss measurements 5 min after FVIII or NaCl 0.9% administration**

| **Product injected** | **Number of animals** | **Blood loss (µl)** | | |
|---|---|---|---|---|
| | | **Mean** | **SE** | **Median** |
| FVIII | 11 | 17.2 | 11.6 | 0 |
| NaCl 0.9% | 19 | 211.7 | 36.3 | 203 |

### C.2. Dose-effect of FVIIa-SR in tail-clip model 5 minutes after injection

Two doses of FVIIa-SR (4 and 8mg/kg) were administered to hemophilia A mice. Results are presented in Table VI and Figure 7. Blood loss measurement was done 5 minutes after products administration. Statistical evaluation of the data showed that administration of 4mg/kg of FVIIa-IR induced a significant decrease in blood loss (P=0.043) compared with negative control group. In contrast, a non-significant reduction and no effect on blood loss (P=0.731) were observed with FVIIa-SR at the same dose (4mg/kg). However, administration of 8mg/kg of FVIIa-SR induced a significant decrease in blood loss (P=0.028) compared with negative control group. Based on these data, the ability of FVIIa-SR to induce a reduction of blood loss in hemophilia A mice in the tail-clip model was further evaluated at the dose of 8mg/kg. In the figure 7, the administered cases of FVIIa-SR and FVIIa-IR are illustrated as F7-SR and F7-IR respectively.

**Table VI: blood loss measurements 5 minutes after FVIIa-IR and FVIIa-SR administration.**

| **Product injected** | **Number of animals** | **Blood loss (µl)** | | |
|---|---|---|---|---|
| | | **Mean** | **SE** | **Median** |
| FVIIa-IR (4mg/kg) | 10 | 88.4 | 35.9 | 65 |
| FVIIa-SR (4 mg/kg) | 10 | 258.0 | 72.5 | 186 |
| FVIIa-SR (8 mg/kg) | 10 | 45.4 | 39.9 | 0 |

### C.3. Prolonged effect of FVIIa-SR in tail-clip model after 120 minutes injection

To evaluate and compare the ability of both FVIIa-SR and FVIIa-IR to induce a significant decrease in blood loss 120 min after administration, both molecules were administered at 8mg/kg to hemophilia A mice. Results are presented in Table VII and Figure 7. Statistical evaluation of the data showed that administration of FVIIa-IR had no effect in blood loss compared with negative control group (P=0.856). In contrast, a significant reduction (P=0.005) on blood loss was observed with FVIIa-SR at the same dose of 8mg/kg compared with negative control group.

**Table VII: Blood loss measurements 120 min after FVIIa-IR and FVIIa-SR administration.**

| **Product injected** | **Number of animals** | **Blood loss (µl)** | | |
|---|---|---|---|---|
| | | **Mean** | **SE** | **Median** |
| FVIIa-IR (8mg/kg | 15 | 268.1 | 88.8 | 168 |
| FVIIa-SR (8 mg/kg) | 18 | 68.5 | 30.8 | 37 |

### C.4. Summary of the results

FVIIa-SR showed a significant ability to correct blood loss in an *in vivo* tail-clip model in hemophilia A mice. 120 minutes, as well as 5 minutes, after its administration at 8mg/kg, this product can significantly reduce blood loss whereas in the same conditions FVIIa-IR cannot.

## Claims

1. Pharmaceutical composition comprising factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the goup consisting of histidine, lysine, aspartic acid and glutamic acid residues, wherein a part of said amino acid residues is substituted with a hydrophobic group.

2. Pharmaceutical composition according to Claim 1, comprising factor VII encapsulated in micelles formed from a copolymer of formula (I) below: 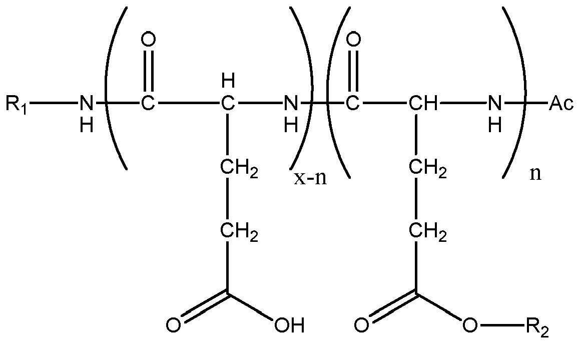 in which:
- R1 signifies a polyalkylene glycol chain,
- R2 is a group chosen from (i) an alkyl chain containing from 5 to 18 carbon atoms and (ii) a benzyl group,
- x is an integer ranging from 10 to 100,
- n is an integer ranging from 1 to x-1, and
- the subunit having the hydrophobic group in the side chain and the subunit having the carboxylic acid group in the side chain are randomly distributed throughout the hydrophobic polymer segment.

3. Pharmaceutical composition according to Claim 2, **characterized in that**, in the copolymer of formula (I), the R1 group signifies a polyethylene glycol chain.

4. Pharmaceutical composition according to either of Claims 2 and 3, **characterized in that**, in the copolymer of formula (I), the R2 group is an unsubstituted alkyl chain containing 8 carbon atoms.

5. Pharmaceutical composition according to one of Claims 2 to 4, **characterized in that**, in the copolymer of formula (I), x is equal to 40.

6. Pharmaceutical composition according to one of Claims 2 to 5, **characterized in that**, in the copolymer of formula (I), the value of n/x is at least 0.1 and less than 1.0, and is preferably 0.9.

7. Pharmaceutical composition accordi n g to one of Claims 1 to 6, **characterized in that** the polyalkylene glycol chain has a molecular weight ranging from 8 kDa to 15 kDa, preferably from 10 kDa to 12 kDa.

8. Pharmaceutical composition according to one of Claims 1 to 7, **characterized in that** the mass ratio between the factor VII and the copolymer ranges from 1:100 wt:wt to 50:100 wt:wt, preferably from 5:100 wt:wt to 20:100 wt:wt.

9. Pharmaceutical composition according to one of Claims 1 to 8, **characterized in that** the factor VII is chosen from (i) a nonrecombinant factor VII purified from plasma and (ii) a recombinant factor VII.

10. Pharmaceutical composition according to one of Claims 1 to 9, **characterized in that** the factor VII is a recombinant human factor VII comprising, as appropriate, the substitution or the deletion of an amino acid.

11. Pharmaceutical composition according to one of Claims 1 to 10, **characterized in that** it is in liquid form or in lyophilized form.

12. Pharmaceutical composition according to one of Claims 1 to 11, **characterized in that** it comprises a buffer for adjusting the pH to a value ranging from 4.5 to 6.5, preferably ranging from 5 to 6.

13. Pharmaceutical composition comprising factor VII encapsulated in micelles formed from block copolymer molecules containing (i) a hydrophilic polymer segment and (ii) a hydrophobic polymer segment consisting of a polyamino acid, which composition, when it is administered parenterally, is suitable for generating a pharmacokinetic profile having one or more of the following characteristics:
- an AUC (area under the curve) value which is of 10% or more higher than the AUC value obtained with a comparative composition wherein the same amount of FVII is not encapsulated
- an MRT (mean residence time) value of at least 2 hours,
and
- a CL (clearance) value of at most 200ml/kg/h.

14. Factor VII encapsulated in micelles formed from a block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, a part of said amino acid residues is substituted with a hydrophobic group, for use thereof as a medicament.

15. Factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for the treatment of coagulation disorders.

16. Use of a factor VII encapsulated in micelles formed from block copolymer molecules containing (i) a hydrophilic polymer segment consisting of a polyalkylene glycol and (ii) a hydrophobic polymer segment consisting of a polyamino acid, with said polyamino acid comprising exclusively amino acid residues selected from the group consisting of histidine, lysine, aspartic acid and glutamic acid residues, wherein a part of said amino acid residues is substituted with a hydrophobic group, for producing a medicament for the treatment of coagulation disorders.
